# EUROPÄISCHE PATENTSCHRIFT

(11) **EP 1 487 961 B1**
(45) Veröffentlichungstag und Bekanntmachung des Hinweises auf die Patenterteilung: **06.07.2011**
(21) Anmeldenummer: 03708240.1
(22) Anmeldetag: 14.03.2003
(51) Int. Cl.: C12M 1/04

(54) **KULTUR/EXPOSITIONSVORRICHTUNGEN, BAUSATZ FÜR DEN ZUSAMMENBAU EINER SOLCHEN SOWIE VERFAHREN ZUR KULTIVIERUNG UND EXPOSITION VON PROKARYONTEN**
CULTURE/EXPOSURE DEVICES, KIT FOR ASSEMBLING A DEVICE OF THIS TYPE AND METHOD FOR CULTIVATING AND EXPOSING PROKARYOTES
DISPOSITIFS DE CULTURE/EXPOSITION, KIT PERMETTANT SON ASSEMBLAGE, ET PROCEDE DE CULTURE ET D'EXPOSITION DE PROCARYOTES

(30) Priorität: 14.03.2002 DE 10211324
(43) Veröffentlichungstag der Anmeldung: 22.12.2004
(73) Patentinhaber: Mohr, Ulrich, 30559 Hannover (DE)
(72) Erfinder: MOHR, Ulrich, 30559 Hannover (DE); DURST, Franz, 91094 Langensendelbach (DE)
(74) Vertreter: Weiss, Peter
(86) Internationale Anmeldenummer: PCT/EP2003/002711
(87) Internationale Veröffentlichungsnummer: WO 2003/076599

(56) Entgegenhaltungen:
- WO-A-01/70927
- DE-A- 19 801 763
- AUFDERHEIDE MICHAELA ET AL: "Novel approaches for studying pulmonary toxicity in vitro." TOXICOLOGY LETTERS (SHANNON), Bd. 140-141, 11. April 2003 (2003-04-11), Seiten 205-211, XP002255450 ISSN: 0378-4274
- KNEBEL JAN W ET AL: "Development of an in vitro system for studying effects of native and photochemically transformed gaseous compounds using an air/liquid culture technique." TOXICOLOGY LETTERS (SHANNON), Bd. 96-97, Nr. SPEC. ISSUE, August 1998 (1998-08), Seiten 1-11, XP002255451 ISSN: 0378-4274

## Beschreibung

Die Erfindung betrifft Kultur/Expositionsvorrichtungen zum Aufnehmen von Kulturen, einen Bausatz zum Zusammenbau solcher Kultur/Expositionsvorrichtungen sowie ein Verfahren zum Kultivieren von Prokaryonten.

Aus dem Stand der Technik sind Kultur/Expositionsvorrichtungen bekannt, bei denen eine in einem Kulturgefäß enthaltene Zellkultur innerhalb der Kultur/Expositionsvorrichtung mit einem gasförmigen Medium beaufschlagt wird. Als Beispiel werden hierzu die gattungsgemäßen Druckschriften DE 100 140 57 des gleichen Anmelders sowie die EP 1174496 genannt.

Die Kultur/Expositionsvorrichtung des gleichen Anmelders nimmt ein Kulturgefäß zum Aufnehmen von Zellkulturen mit einer konisch sich zum Boden verjüngenden Seitenwand auf. Es ist ferner eine Einrichtung zum Beaufschlagen der Zellkulturen mit einem gasförmigen Medium vorgesehen, mit denen die Zellkulturen beispielsweise vorgegebenen schädigenden oder therapeutischen Bedingungen ausgesetzt werden können. Hierzu werden beispielsweise Gase, Aerosole und/oder partikuläre Wirkstoffe unmittelbar mit den Zellkulturen in Kontakt gebracht, wie beispielsweise Tabakrauch auf Lungenzellen. Die Beaufschlagungseinrichtung umfaßt hierzu eine durchgehend zylinderförmige Strömungsführung, die in das Gefäß bis oberhalb der Oberfläche der Zellkultur derart führt, daß ein Strömungsfluß des gasförmigen Mediums durch diese Strömungsführung hindurch, über die Oberfläche der Zellkultur und durch einen zwischen der Strömungsführung und der Kulturbehälterinnenwand entstehenden Ringspalt hindurch erzeugt wird. Eine solche Strömung wird beispielsweise durch eine Vakuumpumpe erzeugt, die in Strömungsrichtung hinter dem genannten Ringspalt angeordnet ist. Die Strömungsführung ist eingangsseitig beispielsweise mit einem Ansaugstutzen verbunden, mit dem Außenluft, Testgase, usw. angesaugt und über die Zellkulturen geleitet werden kann.

Die genannte EP 1174496 entspricht von der Ausgestaltung der Beaufschlagungseinrichtung im wesentlichen der genannten Druckschrift des gleichen Anmelders. Darüber hinaus beschäftigt sich diese Druckschrift damit, die Dosis der auf der Zellkultur abgelagerten Aerosolpartikel, die vom gasförmigen Medium mitgeführt werden, genauer zu bestimmen. Hierzu werden die Stromlinien der Gasströmung innerhalb der zylinderförmigen Strömungsführung durch eine im gepulsten Laserlicht sichtbar werdende markierte Aerosolpartikel ermittelt. Anschließend wird von den ermittelten hyperbelartigen Stromlinien diejenige Stromlinie ermittelt, die in einem bestimmten Abstand oberhalb der Zellkulturoberfläche entlang streicht und bei der die mitgeführten Aerosole noch auf die Zellkulturoberfläche diffundieren können. Diese Stromlinie wird bis zum Eingang der Strömungsführung zurückverfolgt und es wird ein wirksamer Querschnitt innerhalb dieser Stromlinie mit Bezug auf den Gesamtquerschnitt der Öffnung der Strömungsführung ermittelt, durch alle hindurch strömenden Aerosole auf die Zellkulturoberfläche gelangen können.

Es hat sich jedoch in der Praxis herausgestellt, daß die bisherigen Beaufschlagungseinrichtungen unbefriedigend arbeiten, da kein kontinuierlicher Austausch des gasförmigen Mediums oberhalb der Zellkulturoberfläche und auch keine gleichmäßig konzentrierte Beaufschlagung der Zellkulturen gewährleistet werden konnte. Offensichtlich haben sich über der Zellkulturoberfläche stehende "Totzonen" gebildet, in den "verbrauchtes gasförmiges Medium" akkumuliert wurde, innerhalb denen den Zellkulturen also nicht ständig frisches gasförmiges Medium zugeführt werden kann.

Auch haben sich beim Ansaugstutzen Probleme ergeben, da beispielsweise das Absaugen von Raumluft über herkömmliche zylinderförmige Ansaugstutzen starken Schwankungen abhängig von der eingestellten Absaugleistung unterworfen war. Damit ließ sich aber keine raumspezifische Absaugung realisieren, sondern lediglich eine unerwünschte von der Absaugungsleistung abhängige Absaugung.

Der Erfindung liegt die Aufgabe zugrunde, die Strömungseigenschaften des über die Oberfläche einer Kultur geleiteten Gasströmung zu optimieren. Ebenfalls liegt der Erfindung die Aufgabe zugrunde, die Absaugung von gasförmigem Medium beispielsweise aus einem Außenraum zu verbessern. Schließlich liegt ihr auch die Aufgabe zugrunde, bei der Kultivierung und Exposition von Prokaryonten erweiterte Untersuchungsmöglichkeiten zu bieten.

Die Erfindung löst diese Aufgabe jeweils mit den Gegenständen der Ansprüche 1, 18, 25, 26 und 27. Bevorzugte Ausführungsbeispiele der Erfindung sind in den Unteransprüchen beschrieben.

Nach Anspruch 1 ist eine Kultur/Expositionsvorrichtung zum Aufnehmen von Kulturen geschaffen, mit einer Einrichtung zum Beaufschlagen der aufgenommenen Kultur mit einem gasförmigen Medium, die eine mechanische Strömungsführung zum Zwangführen des gasförmigen Mediums mit einem Eingang zum Einleiten des gasförmigen Mediums in die Strömungsführung und einer oberhalb der Oberfläche der Kultur mündenden Ausgangsmündung, die eine sich in Strömungsrichtung trompetenförmig öffnende Innenfläche umfaßt. Der Vorteil dieser trompetenförmig sich öffnenden Ausgangsmündung liegt darin, daß insbesondere schwer zu kontrollierende Ablösezonen an deren Mündungskante vermieden werden, die beispielsweise zu in sich geschlossenen Wirbeln und damit Totzonen oberhalb der Kulturoberfläche führen. Mit dieser speziell geformten Ausgangsmündung wird sichergestellt, daß die Strömung des gasförmigen Mediums glatt und ablösefrei über die Kulturoberfläche geführt wird und die Strömung, einschließlich etwaig darin enthaltener Partikel, etc., über der Kulturoberfläche möglichst gleichmäßig verteilt ist.

Es sei bemerkt, daß mit dem Begriff "trompetenförmig" nicht nur kreisförmige Öffnungen gemeint sind, sondern auch beispielsweise Öffnungen mit einem länglichen (rechteckigen), quadratischen, mehreckigen (rotationssymmetrischen) oder sonstigem Öffnungsquerschnitt. Entscheidend ist lediglich, daß sich der Öffnungsquerschnitt insgesamt zur Mündungskante hin ständig vergrößert, wobei eine "lineare" Vergrößerung in Form einer kegelstumpfartigen Ausgangsmündung noch nicht den gewünschten Erfolg bringt; erst recht keine "tulpenförmige" Ausgangsmündungen. Außerdem umfaßt der allgemeine Begriff "Kultur" sowohl Zellkulturen (oder allgemeiner Eukaryonten-Kulturen) wie auch Prokaryonten-Kulturen (Bakterienkulturen, etc.).

Nach Anspruch 18 ist eine Kultur/Expositionsvorrichtung zum Aufnehmen von Kulturen mit einer Einrichtung zum Beaufschlagen der aufgenommenen Kultur mit einem gasförmigen Medium geschaffen, die einen Ansaugstutzen mit einer Ansaugmündung zum Ansaugen des gasförmigen Mediums und einen Ausgang aufweist, der mit einer Strömungsführung zum Führen des gasförmigen Mediums bis oberhalb der Oberfläche der Kultur verbunden ist, wobei die Ansaugmündung des Ansaugstutzens eine sich gegen die Strömungsrichtung trompetenförmig öffnende Innenfläche aufweist. Hier gilt analog das zum Anspruch 1 Ausgeführte, nämlich daß mit der trompetenförmig sich öffnenden Ansaugmündung insbesondere sich oberhalb des Ansaugstutzens bildende Totzonen vermieden werden, aus denen keine frische Außenluft angesaugt wird.

Nach Anspruch 25 ist ein Bausatz für den Zusammenbau einer Kultur/Expositionsvorrichtung geschaffen, der vier Bausatzelemente enthält, nämlich Aufsätze nach Anspruch 17, Aufsätze mit einer Einrichtung zum Beaufschlagen der in einem Kulturbehälter aufgenommenen Kultur mit einem gasförmigen Medium, wobei die Beaufschlagungseinrichtung einen Ansaugstutzen zum Ansaugen des gasförmigen Mediums und eine mit dem Ansaugstutzen verbundene Strömungsführung zum Führen des gasförmigen Mediums bis oberhalb der Oberfläche der Kultur umfaßt, Untersätze nach Anspruch 12 , sowie Untersätze nach einem der Ansprüche 14 bis 16. Somit lassen sich prinzipiell vier unterschiedliche Kultur/Expositionsvorrichtungen zusammenstellen, eine erste mit einem Untersatz zum Kultivieren von Zellkulturen (oder allgemeiner von Eukaryonten-Kulturen), der insbesondere eine submerse und basale Ernährung der Zellkulturen mit Nährflüssigkeit erlaubt, zusammen mit einem Aufsatz mit der erfindungsgemäßen Strömungsführung, eine zweite mit dem letztgenannten Untersatz zusammen mit einem Aufsatz mit einer "einfacheren" Strömungsführung (z.B. mit durchgehend zylindrischer Strömungsführung), eine dritte mit einem Untersatz zum Kultivieren von Prokaryonten, insbesondere von Bakterien in einer Petrischale (ohne externe Versorgung mit Nährflüssigkeit), zusammen mit einem Aufsatz mit der erfindungsgemäßen Strömungsführung sowie eine vierte mit dem letztgenannten Untersatz zusammen mit einem Aufsatz mit einer "einfacheren" Strömungsführung.

Nach Anspruch 26 ein Verfahren zum Kultivieren von Prokaryonten unter Verwendung einer Kultur/Expositionsvorrichtung mit einer Aufnahme zum Aufnehmen eines Kulturbehälters mit den zu kultivierenden Prokaryonten sowie einer Einrichtung zum Beaufschlagen der im Kulturbehälter aufgenommenen Prokaryonten mit einem gasförmigen Medium geschaffen, wobei die Beaufschlagungseinrichtung einen Ansaugstutzen zum Ansaugen des gasförmigen Mediums und eine mit dem Ansaugstutzen verbundene Strömungsführung zum Führen des gasförmigen Mediums über die im Kulturbehälter aufgenommenen Prokaryonten umfaßt. Hiermit ist vorteilhaft erstmals ein Kultur/Expositionsverfahren geschaffen, bei dem auch Prokaryonten (z.B. Bakterien, Pilze, usw.) einem vorgegebenem gasförmigen Medium ausgesetzt werden können und deren Reaktion darauf untersucht werden kann. Bislang wurden solche Untersuchungen nur an Eukaryonten (Säugetierzellen) durchgeführt. Prokaryonten, oder spezieller Bakterien wurden nur zusammen mit einem flüssigen Wirkstoff beispielsweise in Agar eingekapselt kultiviert und untersucht. Schließlich betrifft der Anspruch 27 eine Kultur/Expositionvorrichtung zum Durchführen eines Verfahrens nach Anspruch 26 mit einer Aufnahme zum Aufnehmen eines Kulturbehälters mit den zu kultivierenden Prokaryonten sowie einer Einrichtung zum Beaufschlagen der im Kulturbehälter aufgenommenen Prokaryonten mit einem gasförmigen Medium geschaffen, wobei die Beaufschlagungseinrichtung einen Ansaugstutzen zum Ansaugen des gasförmigen Mediums und eine mit dem Ansaugstutzen verbundene Strömungsführung zum Führen des gasförmigen Mediums über die im Kulturbehälter aufgenommenen Prokaryonten umfaßt.

Die Erfindung sowie weitere Merkmale und Vorteile der Erfindung werden nunmehr anhand bevorzugter Ausführungsbeispiele mit bezug auf die beigefügte Zeichnung näher erläutert, in der:
- Fig. 1: eine schematischer Längsschnitt durch eine erfindungsgemäße Kultur/Expositionsvorrich- tung mit erfindungsgemäßer Strömungsführung ist,
- Fig. 2: eine schematischer Seitenschnitt durch eine erfindungsgemäße Kultur/Expositionsvorrich- tung mit erfindungsgemäßer Strömungsführung ist,
- Fig. 3: eine schematische Aufsicht auf den Unter- satz einer erfindungsgemäßen Kultur/Exposi- tionsvorrichtung ist,
- Fig. 4a - c: eine Schnittansicht, eine perspektivische Ansicht von schräg oben bzw. eine perspek- tivische Ansicht von schräg unten einer er- findungsgemäßen Strömungsführung ist,
- Fig. 5a und b: eine schematische Aufsicht auf bzw. eine schematische Seitenansicht einer in der er- findungsgemäßen Kultur/Expositionsvorrich- tung verwendeten Ringblende zum gleichmäßi- gen Verteilen der Ansaugströmung um die Strömungsführung ist,
- Fig. 6a und b: eine schematische Seitenansicht bzw. eine Aufsicht auf einen in der erfindungsgemäßen Strömungsführung angeordneten Drallkörper sind,
- Fig. 7: eine schematische Schnittansicht durch die Anordnung von erfindungsgemäßer Strömungs- führung und Kulturbehälter mit aufgenomme- ner Kultur ist,
- Fig. 8a und b: eine schematische Schnittansicht eines er- findungsgemäßen Ansaugstutzens gemäß einem ersten Ausführungsbeispiel bzw. eine sche- matische Aufsicht auf einen Bodenbereich dieses erfindungsgemäßen Ansaugstutzens sind,
- Fig. 9a und b: eine schematische Schnittansicht eines er- findungsgemäßen Ansaugstutzens gemäß einem zweiten Ausführungsbeispiel bzw. eine sche- matische Aufsicht auf einen Bodenbereich dieses erfindungsgemäßen Ansaugstutzens sind,
- Fig. 10: eine perspektivische Schrägansicht eines weiteren Aufsatzes für den erfindungsgemä- ßen Bausatz zum Zusammenbau einer Kul- tur/Expositionsvorrichtung ist,
- Fig. 11: eine perspektivische Seitenansicht eines weiteren Untersatzes für die Aufnahme von Zellkulturbehältern mit einer Versorgungs- einrichtung zum Versorgen der Zellkulturen mit einem Flüssigmedium ist, der ein Bausatzteil des erfindungsgemäßen Bausatzes zum Zusammenbau einer Kultur/Expositions- vorrichtung ist, und
- Fig. 12: eine perspektivische Seitenansicht einer aus dem Aufsatz der Fig. 10 und dem Unter- satz der Fig. 11 aufgebauten Kultur/Exposi- tionsvorrichtung ist.

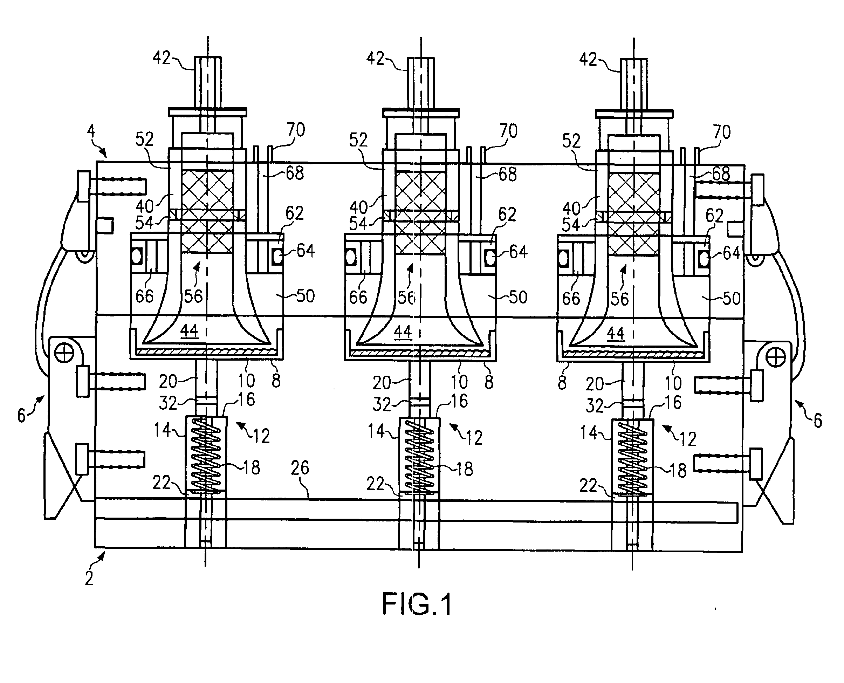

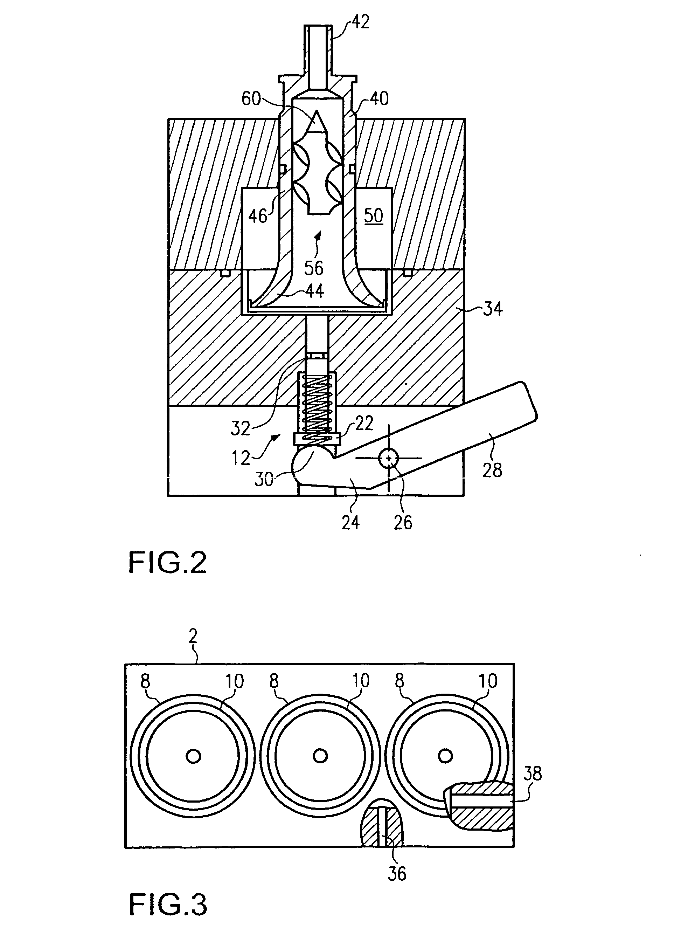

Fig. 1 zeigt ein schematische Längsschnitt durch eine erfindungsgemäße Kultur/Expositionsvorrichtung. Diese hat nahezu Kastenform und ist aus zwei Kastenhälften, einem Untersatz 2 und einem Aufsatz 4 zusammengesetzt. Im zusammengesetzten Zustand können Unter- 2 und Aufsatz 4 über einen Verschluß 6 in Form eines Schnallenmechanismus verschlossen werden. Der Untersatz 2 enthält in dem in Fig. 1 gezeigten Beispiel drei Aufnahmen 8 für die Aufnahme von drei Kulturbehältern 10. Selbstverständlich kann diese Anzahl beliebig gewählt werden. Die Aufnahmen 10 sind zylindrische Ausnehmungen in dem blockförmigen Untersatz 2, was aus der Aufsicht auf den Untersatz in Fig. 3 ersichtlich wird. Auch die Kulturbehälter 10 haben eine kreisrunde Becherform mit zylindrischen Außenwänden, wobei die Höhe des Bechers etwas niedriger als die Tiefe der zylindrischen Ausnehmungen für die Aufnahmen 8 ist.

Zum Entnehmen des Kulturbehälters 10 ist ein Auswurfmechanismus 12 vorgesehen, der besonders deutlich in der Schnittansicht der Fig. 2 ersichtlich wird. Der Auswurfmechanismus umfaßt eine zylindrische Durchgangsbohrung 14, die von der Unterseite des Untersatzes 2 bis in die Aufnahme 8 reicht, mit einem - von der Unterseite des Untersatzes 2 her gesehenen - Vorsprung 16, an dem sich eine Rückstellfeder 18 abstützt. In der Durchgangsbohrung 14 ist ein Auswurfbolzen 20 hin- und herbeweglich geführt, dessen Außendurchmesser etwa dem Innendurchmesser der Durchgangsbohrung 14 in deren verjüngtem Abschnitt entspricht. Der Auswurfbolzen 20 weist einen zylindrischen Kopf 22 auf, dessen Außendurchmesser dem Innendurchmesser der Durchgangsbohrung 14 in deren erweitertem Abschnitt entspricht. Hiermit ist eine exakte Führung des Auswurfbolzens 20 in der Durchgangsbohrung 14 gewährleistet. An der Unterseite des Kopfes 22 greift die Rückstellfeder 18 an, die den Auswurfbolzen 20 somit in Richtung von der Aufnahme 8 weg vorspannt. An der flachen Außenseite des Kopfes 22 greift ein Hebel 24 an, der um eine von der Durchgangsbohrung 14 versetzte Schwenkachse 26 in Längsrichtung des Auswurfbolzens 20 schwenkbar ist. Der Hebel 24 ragt von der Unterseite des Untersatzes 2 in Form eines manuell betätigbaren Griffstükkes 28 aus der Kultur/Expositionsvorrichtung hervor. Wie aus Fig. 1 ersichtlich wird, ist für alle drei Auswurfmechanismen 12 eine gemeinsame Schwenkachse 26 in Form eines in Längsrichtung durch den Untersatz 2 hindurch ragenden Durchgangsbolzen vorgesehen. Dieser Durchgangsbolzen ist im Untersatz 2 festgelegt, während die Hebel 24 um ihn frei schwenkbar angeordnet sind. Somit ragen aus der Längsseite des Untersatzes 2 drei Griffstücke 28 hervor, mit denen die drei Kulturbehälter 10 einzeln manuell ausgeworfen werden können. Der Hebel 24 weist einen abgerundeten Betätigungsabschnitt 30 auf, der an der Stirnfläche des Kopfes 22 anschlägt. Mit diesem abgerundeten Betätigungsabschnitt 30 wird die Schwenkbewegung des Hebels 24 möglichst reibungsfrei in die Hin- und Herbewegung des Auswurfbolzens 20 übertragen. Der Auswurfbolzen 20 weist in Höhe des verjüngten Abschnitts der Durchgangsbohrung 14 eine Ringdichtung 32 auf, die die Aufnahme 8 vom unteren Abschnitt des Auswurfmechanismus 12 abdichtet. Selbstverständlich ist auch jede andere Art von Auswurfmechanismus möglich, mit der der Kulturbehälter 10 für eine leichtere Entnahme aus seiner Aufnahme 8 ausgeworfen werden kann.

Der Untersatz 2 ist im wesentlichen hohl und bildet um die Aufnahme 8 herum eine flüssigkeitsdichte Kammer 34, in die eine Flüssigkeit zum Temperieren der Aufnahme 8 und damit der im Kulturbehälter 10 aufgenommenen Kultur gefüllt ist. Für die Temperatursteuerung ist ferner ein Flüssigkeitseinlaß 36 und ein Flüssigkeitsablaß 38 (in Fig. 3 ersichtlich) an der Kammer 34 vorgesehen, die mit einem externen Heizkreislauf zum Einstellen der Temperatur der Flüssigkeit in der Kammer 34 verbunden werden können. Es kann selbstverständlich eine alternative Heizeinrichtung um die Aufnahme 8 herum vorgesehen sein, beispielsweise eine Heizwicklung, etc., mit der der Kulturbehälter 10 auf einer vorgegebenen Temperatur aufbewahrt werden kann.

Der Aufsatz 4 umfaßt eine Beaufschlagungseinrichtung zum Beaufschlagen der in dem Kulturbehälter 10 aufgenommenen Kultur mit einem gasförmigen Medium. Das gasförmige Medium kann beispielsweise als reines Gas vorliegen, d.h. alle darin enthaltenen Stoffe, (Atome, Moleküle etc.) befinden sich in der Gasphase, es kann auch als Träger für Fest-und/oder Flüssigstoffe dienen und/oder es kann als Gasgemisch vorliegen (wenn es beispielsweise darum geht, gasförmige Bestandteile auf die Kulturen aufzubringen). Insbesondere können so Aerosole, zerstäubte Flüssigkeiten, kleine Flüssigkeitströpfchen (z.B. Pflanzenschutzmittel als Sprühnebel, etc.) Schwebeteilchen, Feststoffpartikel (z.B. Holzstaub etc.) gasförmige Suspensionen, zerstäubte Suspensionen oder Emulsionen als zu tragende Substanzen in dem Trägergas enthalten sein. Beispielsweise kann die Belastung von Lungenzellen mit Zigarettenrauchpartikeln untersucht werden. Die aufgezählten Stoffe sind nicht abschließend, sondern können je nach Experiment weiter variieren.

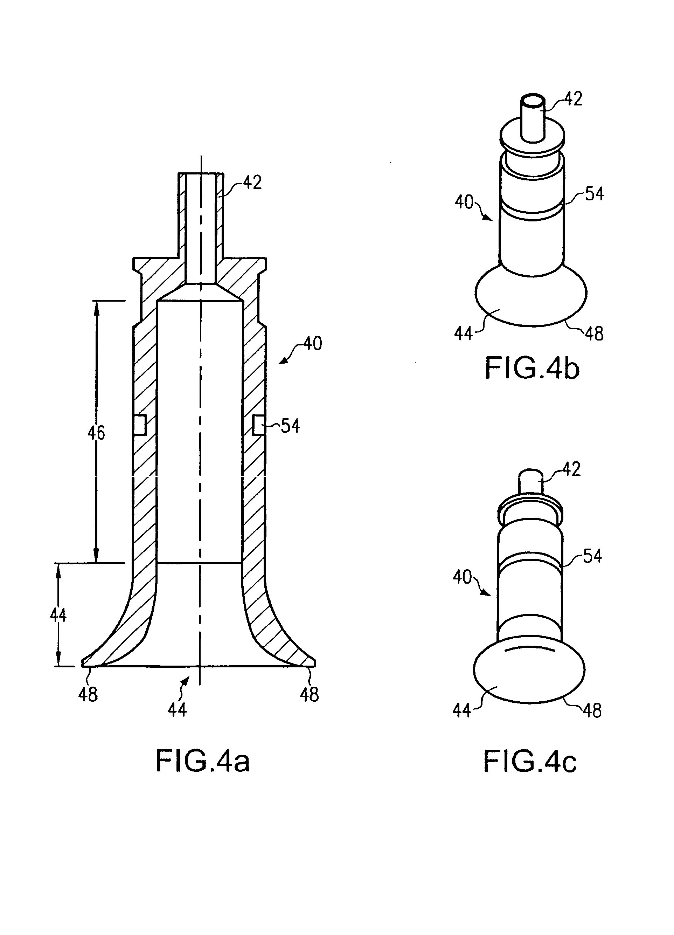

Die Beaufschlagungseinrichtung umfaßt eine Strömungsführung 40 mit einem Eingang 42 in Form eines Anschlußstutzens und einer Ausgangsmündung 44, die bei auf dem Untersatz 2 aufgesetzten Aufsatz 4 knapp oberhalb der Oberfläche der Kultur im Kulturbehälter 10 mündet. Die Strömungsführung 40 umfaßt einen zylindrischen Führungsabschnitt 46 mit einer zylindrischen Innenbohrung, die stetig in die sich in Strömungsrichtung trompetenförmig öffnende Innenfläche der Ausgangsmündung 44 übergeht. Diese Innenfläche ist in Strömungsrichtung bevorzugt hyperboloidförmig (siehe insbesondere Figuren 4a-c). Die Außenform der Strömungsführung 40 ist von der hyperboloidförmigen Innenfläche unabhängig. Je nach Anwendung (Zusammensetzung des Aerosols, etc.) können besondere Gestaltungen der Mündungskante 48 der Ausgangsmündung 44 vorteilhaft sein (z.B. Abrundungen, möglichst scharfe Kante, etc.). Die Innenfläche der Ausgangsmündung 44 verläuft an der Mündungskante 48 bereits horizontal, d.h. daß die Hyperbel der geschnittenen Innenfläche so ausgebildet ist, daß sie an der Mündungskante 48 eine horizontale Steigerung besitzt. Insgesamt führt der Hyperbelverlauf also vom in den zylindrischen Führungsabschnitt 46 übergehenden vollständig senkrechten Verlauf in den an der Mündungskante 48 vorliegende vollständig horizontalen Verlauf, womit die an der Innenfläche der Strömungsführung 40 anliegende Strömung somit um einen 90° Winkel radial nach außen umgelenkt wird. Diese spezielle Geometrie ermöglicht eine glatte ablösungsfreie Strömung, die unter anderem die kontinuierliche Zufuhr von frischem gasförmigen Medium auf die Kulturoberfläche sicherstellt. Darüber hinaus ist die Konzentrationsverteilung des Mediums über der zu beaufschlagenden Oberfläche nahezu völlig gleichmäßig.

Die Strömungsführung 40 ist in einer im Aufsatz 4 von dessen Oberseite in einen Innenraum 50 mündenden Durchgangsbohrung 52 reibschlüssig gehalten. In der Außenfläche des zylindrischen Führungsabschnitts 46 ist eine Ringnut 54 zur Aufnahme einer Ringdichtung vorgesehen, die somit zwischen der Außenwand der Strömungsführung 40 und der Innenwand der Durchgangsbohrung 52 den Außenraum der Kultur/Expositionsvorrichtung gegen den Innenraum 50 luftdicht abdichtet. Die Strömungsführung 40 ist in der Durchgangsbohrung 52 in Längsrichtung verschiebbar, damit der Abstand der Mündungskante 48 zur Oberfläche der in dem Kulturbehälter 10 aufgenommenen Kultur eingestellt werden kann (siehe nähere Diskussion unten).

Der Innenraum 50 bildet im zusammengesetzten Zustand von Unter- 2 und Aufsatz 4 mit der Aufnahme 8 einen geschlossenen Innenraum von zylindrischer Form, wobei der Innenraum 50 und die Aufnahme 8 stoßkantenfrei ineinander übergehen.

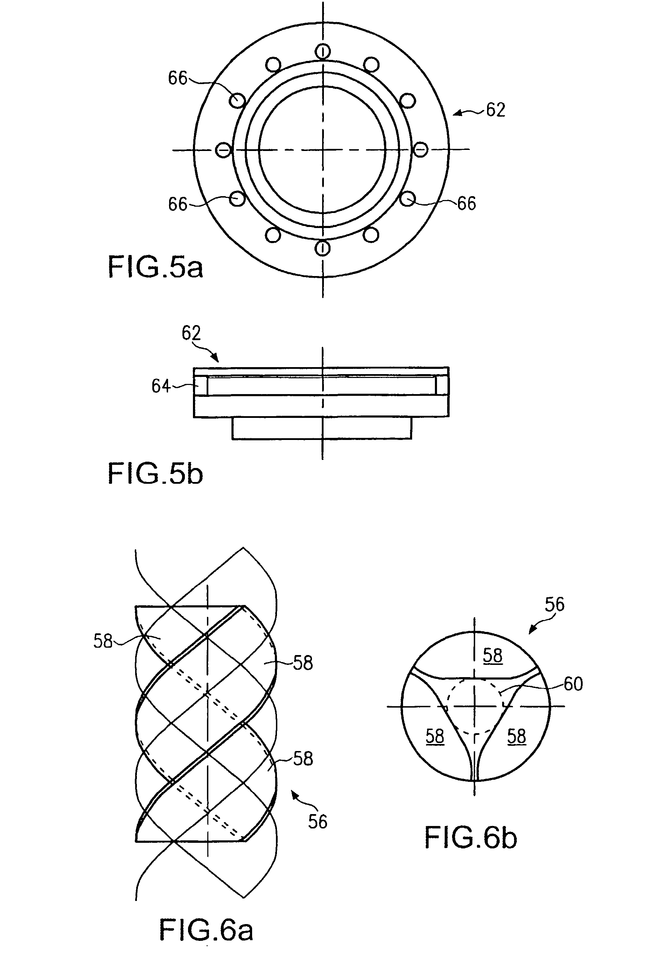

Im zylindrischen Führungsabschnitt 46 ist ein in den Fig. 6a und b näher dargestellter Drallkörper 56 reibschlüssig aufgenommen. Der Drallkörper 56 ist ein kurzes zylinderförmiges Formstück, dessen Außendurchmesser dem Innendurchmesser des zylindrischen Führungsabschnitts 46 entspricht und in den drei nebeneinander angeordnete schneckenförmig verlaufende Rillen 58 angeordnet sind. Selbstverständlich ist die Anzahl der nebeneinander angeordneten Rillen 58 beliebig. Die Länge der Rillen 58 bezüglich der Länge des gesamten Drallkörpers 56 ist derart, daß die Rillen 58 im wesentlichen eine halbe Umdrehung um den zylinderförmigen Drallkörper 56 herum durchführen. Ferner sind die Rillen 58 so dicht nebeneinander angeordnet, daß der dazwischen liegende verbleibende Steg möglichst dünn ist, insgesamt also der Durchflußquerschnitt aller Rillen 58 möglichst groß ist. Zentral an der Oberseite des Drallkörpers 56 ist eine Kegelspitze 60 ausgebildet, deren Außenwandung an ihrer Basis stoßkantenfrei in den Boden der Rillen 58 übergehen. Die Kegelspitze 60 ist gegen die Strömung gerichtet. In diesem Zusammenhang ist der Begriff "stoßkantenfrei" so gewählt, daß zwar ein gegebenenfalls mehr oder weniger scharfer Knick zwischen Außenfläche der Kegelspitze 60 und Innenfläche der Rillen 58 an deren tiefster Stelle vorliegt, jedoch keine Versetzung, an der sich anderenfalls möglicherweise Wirbelablösungen der Strömung einstellen können. In Fig. 6b ist die kreisförmige Basisfläche der Kegelspitze 60 gestrichelt eingezeichnet.

Um den zylindrischen Führungsabschnitt 46 der Strömungsführung 40 ist ferner eine in Längsrichtung verschieblich geführte scheibenförmige Ringblende 62 angeordnet, deren Innendurchmesser dem Außendurchmesser des zylindrischen Führungsabschnitts 46 und deren Außendurchmesser dem Innendurchmesser des Innenraum 50 im Aufsatz 4 entspricht. An der zylinderförmigen Außenwand der Ringblende 62 ist ferner eine Ringnut 64 zur Aufnahme eines Dichtungsringes eingelassen, der die zylindrische Außenwand der Ringblende 62 gegen die Innenwand des Innenraums 50 abdichtet. Wie aus Fig. 5a ersichtlich wird, umfaßt die Ringblende 62 mehrere axial verlaufende Durchgangsbohrungen 66, die den von der Ringblende 62 abgetrennten oberen Raumabschnitt des Innenraums 50 mit dem unteren Raumabschnitt des selben kommunizieren lassen. An der Oberseite des Innenraums 50 ist ferner eine Durchgangsbohrung 68 bis zu einem Anschlußstutzen 70 vorgesehen, an den beispielsweise über eine Schlauchverbindung eine Vakuumpumpe angeschlossen werden kann, die den Innenraum 50 somit unter Unterdruck setzt. Beispielsweise kann eine Vakuumpumpe für alle drei an der in Fig. 1 gezeigten Kultur/Expositionsvorrichtung über einen entsprechenden Schlauchverteiler angeschlossen sein. Die Ringblende 62 dient nunmehr der gleichmäßig rotationssymmetrischen Verteilung des Unterdrucks und der sich hierdurch einstellenden Strömung um die gesamte Außenfläche der Strömungsführung 40 herum, um die aufgrund der Anordnung der Durchgangsbohrung 66 bezüglich der Strömungsführung 40 fehlende Rotationssymmetrie auszugleichen (siehe auch Figuren 5a und b). Außerdem können unterschiedliche Ringblenden 62 pro Strömungsführung 40 vorgesehen sein, die sich in der Dimensionierung ihrer Durchgangsbohrungen 66 unterscheiden. Je nach einzustellendem Unterdruck in der Strömungsführung 40 wird eine entsprechende Ringblende 62 ausgewählt und über die Strömungsführung 40 geschoben.

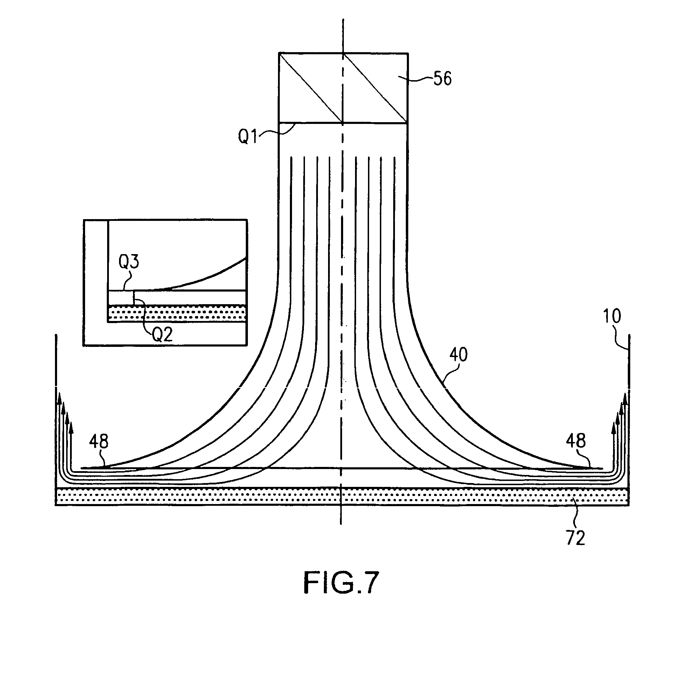

Fig. 7 zeigt eine schematische Schnittansicht durch die Strömungsführung 40 und den Behälter 10 zum Erläutern der Einstellung des Abstandes zwischen der Mündungskante 48 und der Oberfläche einer im Behälter 10 aufgenommenen Kultur 72. Der Abstand der Ausgangsmündung 44 zur Oberfläche der Kultur 72 wird derart eingestellt, daß der Durchflußquerschnitt Q2 des Ringspaltes zwischen der Mündungskante 48 und der Oberfläche der Kultur 72 kleiner als der Durchflußquerschnitt Q1 im zylindrischen Führungsabschnitt 46 der Strömungsführung 40 ist. Mit dieser Maßnahme wird die Strömung über der Oberfläche der Kultur 72 beschleunigt, was wiederum die Entstehung von Wibelablösegebieten vermeidet. In der Regel liegt der Abstand der Mündungskante 48 von der Oberfläche der Kultur 72 im Millimeterbereich, beispielsweise 1 mm. Ein solcher Mindestabstand ist auch deshalb erforderlich, da die Oberfläche der Kultur 72 stellenweise uneben sein kann. Als weitere Maßnahme ist vorgesehen, das Verhältnis des Innendurchmessers von Behälter 10 und Außendurchmesser der Ausgangsmündung 44 an der Mündungskante 48 derart zu dimensionieren, daß der Durchflußquerschnitt Q3 des Ringspalts zwischen Mündungskante 48 und Innenwand des Behälters 10 ebenfalls größer als der zuvor genannte Durchflußquerschnitt Q1 ist. Insgesamt sind der Höhe der Strömungsführung 40 - abgesehen von der Ausbildung der Hyperboloidform der Ausgangsmündung 44 - keine Höhenbeschränkungen auferlegt. Selbstverständlich kann der zylindrische Führungsabschnitt 46 auch weggelassen werden, der Eingang 42 also direkt in die hyperboloidförmige Ausgangsmündung 44 einleiten. Der Durchmesser der Ausgangsmündung 44 an ihrer Mündungskante 48 wird bei Einhaltung des genannten Verhältnisses Q3/Q1 vor allem von der Größe des Kulturbehälters bestimmt, der ein in der Praxis häufig verwendeter Standardbehälter sein kann.

Der Abstand der Mündungskante 48 von der Oberfläche der Kultur 72 kann entweder dadurch eingestellt werden, daß in die Aufnahme 8 vor Einsetzen des Kulturbehälters 10 mit Kultur 72 ein Einstellplättchen mit bekannter Dicke gelegt wird und die Strömungsführung 40 bei geschlossenem Unter- 2 und Aufsatz 4 solange gegen die Reibschlußkraft nach unten gedrückt wird, bis die Mündungskante 48 auf dem Einstellplättchen aufliegt. Anschließend kann die Kultur/Expositionsvorrichtung geöffnet werden, das Einstellplättchen entnommen werden und an dessen Stelle der Kulturbehälter 10 mit der aufgenommenen Kultur 72 eingesetzt werden. Alternativ ist im Deckenbereich des Aufsatzes 4 eine Einstelleinrichtung zum manuellen oder automatischen Verstellen des Abstandes vorgesehen. Die manuelle Einstelleinrichtung kann beispielsweise ein Schneckentrieb mit Rändelschraube als manuelles Betätigungselement sein, wobei der Schneckentrieb zwischen dem Aufsatz 4 und der Strömungsführung 40 wirkt. Im anderen Fall können auch Einstellmarkierungen an dem aus dem Aufsatz 4 hervorstehenden Ende der Strömungsführung vorgesehen sein, mit dem ein oder mehrere verschiedene Abstände eingestellt werden können. Anstelle der reibschlüssigen Verbindung zwischen Strömungsführung 40 und Aufsatz 4 kann auch eine Schraubverbindung vorgesehen sein, so daß der Abstand durch Verdrehen der Strömungsführung 40 gegen den Aufsatz 4 eingestellt werden kann.

Zum Temperieren des gasförmigen Mediums kann eine Heizwicklung um die Strömungsführung 40 vorgesehen sein, die mit einer entsprechenden Heizeinrichtung zum Steuern der Temperatur verbunden ist. Alternativ kann die Strömungsführung 40 aus einem korrisionsbeständigen Metall (vorzugsweise Titan) ausgebildet oder zumindest von diesem ummantelt sein, an das ebenfalls ein Strom zum Erwärmen angelegt werden kann. Weiterhin kann entweder zwischen Eingang 42 der Strömungsführung 40 und einem damit verbundenen Ansaugstutzen oder zwischen den Anschlußstutzen 70 und die Vakuumpumpe ein Volumendurchflußmesser oder Massendurchflußmesser mit einem gekoppelten Stellventil geschaltet sein. Hiermit können beispielsweise die Durchflußmengen des gasförmigen Mediums durch die einzelnen Strömungsführungen 40 individuell eingestellt werden.

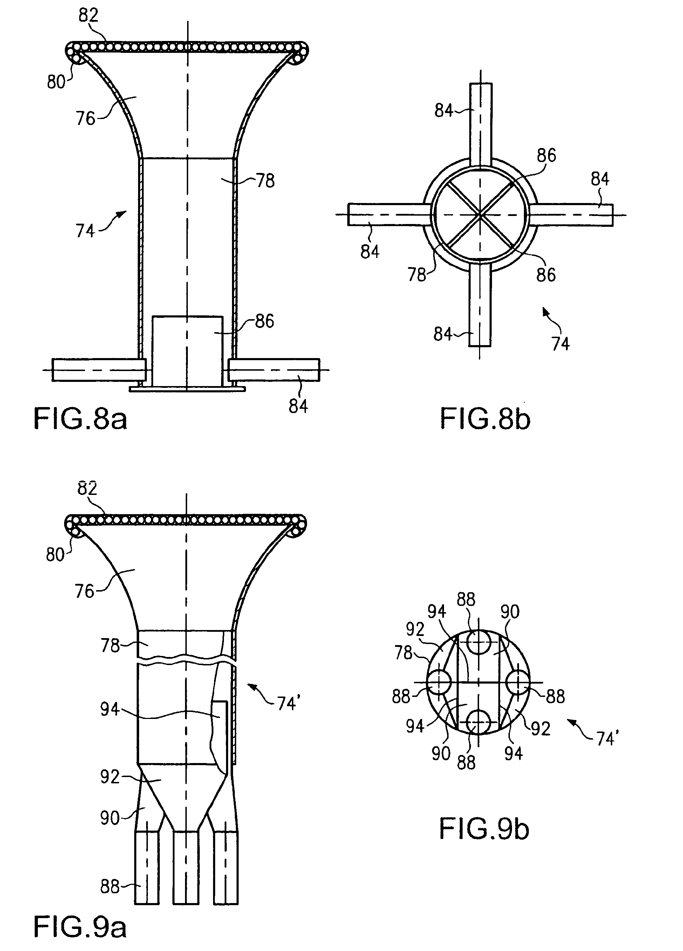

In den Fig. 8a und b bzw. 9a und b sind zwei alternative Ausgestaltungen eines erfindungsgemäßen Ansaugstutzens 74 bzw. 74' dargestellt, der mit den Eingängen 42 der mehreren Strömungsführungen 40 verbunden sein kann. Die Ansaugstutzen 74 bzw. 74' sind im Prinzip ähnlich wie die Strömungsführung 40 aufgebaut, d.h. sie umfassen eine Ansaugmündung 76 mit einer sich gegen die Strömungsrichtung trompetenförmig öffnenden Innenfläche. Die Innenfläche hat insbesondere in Strömungsrichtung eine Hyperboloidform. Ferner schließt sich an diese Innenfläche ein zylindrischer Führungsabschnitt 78 mit einer zylindrischen Innenfläche an. Die Ansaugmündung 76 in Hyperboloidform kann - anders als in den Fig. 8a und 9a gezeigt - sich bevorzugt auch soweit öffnen, daß die Mündungskante 80 einen horizontalen Verlauf hat, somit also wiederum eine Strömungsumlenkung der an der Ansaugmündung 76 anliegenden Strömung um 90° von der Horizontalen in die Vertikale stattfindet. Über die Öffnung der Ansaugmündung 76 ist ein grobporiger Schaumstoff 82 gespannt, der eine gleichmäßige Strömung in die Ansaugmündung 76 selbst bei Zug oder Turbulenzen in der Raumluft sicherstellt und Störungen dämpft, ohne daß er beispielsweise Rauchpartikel oder andere partikuläre Feststoffe bzw. Flüssigstoffe herausfiltert. Er verhindert somit insbesondere, daß seitlich auf den Ansaugstutzen 74, 74' auftreffende Strömungen den eigentlichen Strömungsfluß in die Ansaugmündung 76 hinein stören. Anstelle des aufgespannten Schaumstoffes 82 kann auch ein entsprechend grobmaschiges Gitter in der Ansaugmündung 76 vorgesehen sein oder irgend ein anderes Mittel, das auf den Mündungsbereich der Ansaugmündung 76 treffende Querströmungen dämpft.

Der in den Fig. 8a und b gezeigte Ansaugstutzen 74 weist im Bodenbereich seines zylindrischen Führungsabschnitts 78 radial nach außen gerichtete Anschlußstutzen 84 für den Anschluß von Leitungen zu den einzelnen Eingängen 42 der Strömungsführungen 40 auf. Wie in Fig. 8b ersichtlich, sind die radial vom Führungsabschnitt 78 abstehenden Anschluß-stutzen 84 rotationssymmetrich um dessen Außenwand angeordnet. In dem gezeigten Beispiel sind vier um 90° versetzt angeordnete Anschlußstutzen 84 vorgesehen. Selbstverständlich kann auch eine andere Anzahl an Anschlußstutzen 84, beispielsweise acht, vorgesehen sein. Um Strömungswechselwirkungen der vier sich gegenüberliegenden Anschlußstutzen 84 innerhalb des Führungsabschnitts 78 zu vermeiden, sind darin in deren Höhe Trennwände 86 symmetrisch am Bodenbereich des Führungsabschnitts 78 angeordnet. Sie reichen vom geschlossenen Boden des Führungsabschnitts 78 etwa zweimal so hoch wie die Öffnungen der gleich hoch, nahe dem Boden angeordneten Anschlußstutzen 84. Insgesamt bilden die vier Trennwände 86 vier viertelkreisförmige Kammern. Das Verhältnis von Länge zu Durchmesser des Führungsabschnitts 78 liegt in etwa in der Größenordnung von 2, wobei die Länge der geradlinigen radial abstehenden Anschlußstutzen 84 in etwa dem Durchmesser des Führungsabschnitts 78 entspricht. Der Durchmesser der Anschlußstutzen 84 ist in etwa um den Faktor 10 kleiner als die Länge des Führungsabschnittes 78 und die Höhe der Trennwände 86 beträgt in etwa das dreifache des Durchmessers des Anschlußstutzens 84.

Die in den Fig. 9a und b gezeigte alternative Ausführungsform des Anschlußstutzens 74' weist vier vom Boden des Führungsabschnitts 78 in Längsrichtung abstehende Anschlußstutzen 88 auf, die über entsprechend geformte Übergangsabschnitte 90 und 92 in den Führungsabschnitt 78 mit zylindrischer Innenfläche übergehen. Wie aus den Fig. 9a und b ersichtlich wird, sind die Übergangsabschnitte 90 und 92 zu den Anschlußstutzen 88 nicht rotationssymmetrisch ausgebildet, im Gegensatz zur Anordnung der Anschlußstutzen 88 bezüglich des Bodens des Führungsabschnitts 78. Daher wurden entsprechende Trennwände 94 zum Aufteilen des durch den Führungsabschnitt 78 fließenden Volumenstroms in gleiche Teile auf die vier Anschlußstutzen 88 vorgesehen. Diese umfassen zwei Trennwände 94, welche eine zentrale Verbindungslinie zwischen den beiden Übergangsabschnitten 92 in etwa auf in drei gleiche Strecken aufteilen, und dabei senkrecht zur genannten Verbindungslinie stehen, sowie eine Trennwand 94 entlang der genannten Verbindungslinie, die zwischen den beiden erst genannten Trennwänden 94 angeordnet ist und die Übergangsabschnitte 90 im zylindrischen Führungsabschnitt 80 voneinander trennt. Bei diesem Ansaugstutzen 74' beträgt das Verhältnis von Höhe zu Durchmesser des Führungsabschnitts 78 in etwa fünf, das Verhältnis der Durchmesser von Führungsabschnitt 78 und von Ansaugstutzen 88 etwa vier und das Verhältnis von Höhe der Trennwände 94 (die genannten drei Trennwände sind gleich hoch) zu Höhe des Führungsabschnitts 78 etwa sieben.

Selbstverständlich kann sich ein solcher Ansaugstutzen 74 auch unmittelbar an eine Strömungsführung 40 anschließen, d.h. ohne die Verbindung über seinen Anschlußstutzen 84, die Verbindungsleitung und den Eingang 42 der Strömungsführung 40. Der zylindrische Führungsabschnitt 78 des Ansaugstutzens 74 kann somit direkt bei gleichem Durchmesser in den zylindrischen Führungsabschnitt 46 der Strömungsführung 40 übergehen, oder es kann sogar die trompetenförmige sich öffnende Ansaugmündung 76 des Ansaugstutzens 74 unmittelbar in die trompetenförmig sich öffnende Ausgangsmündung 44 der Strömungsführung 40 übergehen. Der Ansaugstutzen 74, 74' kann entweder in der Außenluft positioniert sein, oder in einem entsprechenden Raum, dem die von dem gasförmigen Medium mitzuführenden Bestandteile künstlich zugegeben werden (beispielsweise werden Flüssigkeitströpfchen über entsprechende Flüssigkeitsdüsen in den Raum gesprüht, oder es werden Feststoffe über eine Öffnung in den Raum geblasen, ein Rauchroboter erzeugt Tabakrauch im Raum, etc.).

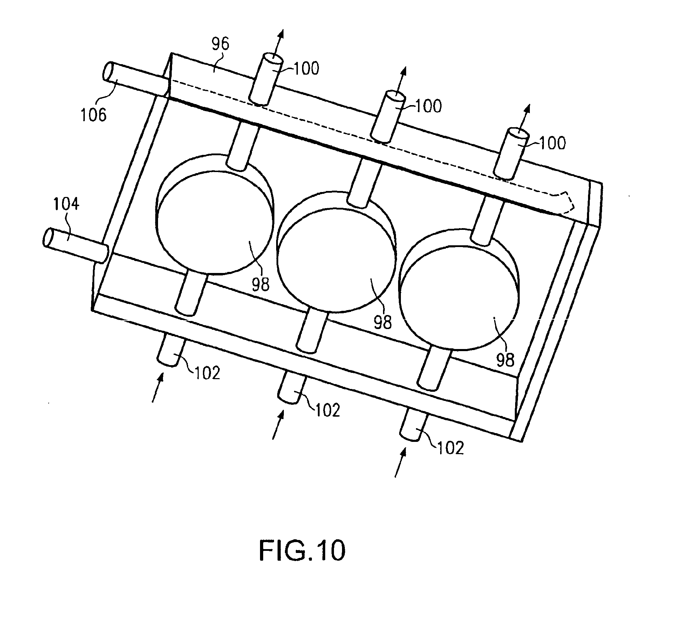

Nachfolgend werden weitere Bestandteile in Form von Auf-und Untersätzen eines Bausatzes für eine Kultur/Expositionsvorrichtung beschrieben, welche in Kombination mit den vorstehend beschriebenen Unter- 2 und Aufsätzen 4 im Bausatz kombiniert werden können. So zeigt Fig. 10 einen Aufsatz 96 mit einer vereinfachten Beaufschlagungseinrichtung zum Beaufschlagen von Kulturen mit einem gasförmigen Medium, die drei im wesentlichen becherförmige Ausnehmungen mit rotationssymmetrischer Innenfläche als Strömungsführungen 98 zum Führen des gasförmigen Mediums bis oberhalb der Oberfläche der Kultur umfaßt. Die Strömungsführungen 98 haben in Höhe ihrer Mündungskante ein zylindrisches Innenprofil, daß abgerundet in einen flachen Bodenbereich übergeht. Von beiden Längsseiten des Aufsatzes 96 ragen Anschlußstutzen 100 und 102 durch den Aufsatz 96 hindurch in die Strömungsführungen 98 und dienen als Zu- und Ablauf für das gasförmige Medium in die Strömungsführungen 98. Das gasförmige Medium gelangt somit beispielsweise über einen als Zulauf fungierenden Anschlußstutzen 102 (der beispielsweise mit dem erfindungsgemäßen Ansaugstutzen 74, 74' verbunden ist) an einer Seitenwand in die Strömungsführung 98 und wird an der gegenüberliegenden Seitenwand der Strömungsführung 98 über den als Ablauf fungierenden Anschlußstutzen 100 (der beispielsweise mit einer Vakuumpumpe verbunden sein kann) aus der Strömungsführung 98 abgesaugt. Hierbei handelt es sich um eine in strömungstechnischer Hinsicht gesehen einfacher gestalteten Strömungsführung 98, die nicht die gleiche zeitlich und räumlich homogene Verteilung des gasförmigen Mediums über der Gesamtfläche der Ausgangsmündung der Strömungsführung 98 hat. Ferner umfaßt der als flüssigkeitsdichter Hohlkörper ausgebildete Aufsatz 96 noch einen Zu- 104 und Ablauf 106 für eine Heizflüssigkeit, mit der die Strömungsführungen 98 und damit das gasförmige Medium oberhalb der Kultur temperiert werden können.

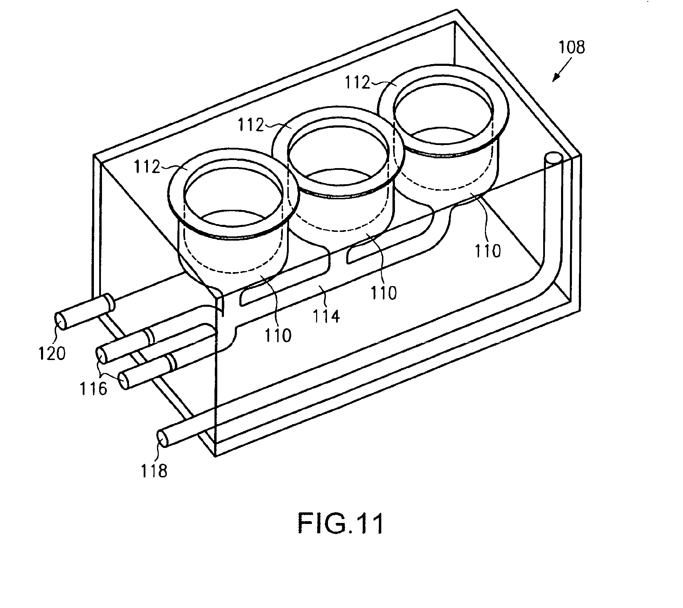

Fig. 11 zeigt einen weiteren Untersatz 108, der für die Aufnahme von Kulturbehältern mit darin enthaltenen Zellkulturen (allgemeiner Eukaryonten) ausgebildet ist. Hierzu enthält der Aufsatz 108 drei Aufnahmen 110 für die Aufnahme von drei Kulturbehältern 112 (z.B. Transwell-Inserts), in denen die Zellkulturen aufgenommen sind. An dieser Stelle wird auf das deutsche Patent 198 017 63 verwiesen, das eine Kulturvorrichtung zum Kultivieren von Zellkulturen offenbart. Die Offenbarung dieses Patentes wird hiermit durch Bezugnahme vollinhaltlich in die vorliegende Anmeldung mit aufgenommen.

Diese Kulturbehälter 112 (Transwell-Inserts) haben beispielsweise eine becherartige Form mit kreisförmigen Querschnitt, wobei sich der Durchmesser von der Becheröffnung bis zum Becherboden konisch verjüngt. Der Becherboden besteht aus einem porösen Kunststoffmaterial, z.B. aus Polyethylenrephthalat. Das Zellkulturinsert stellt eine flüssigkeitsdurchlässige Tragstruktur für eine Membran dar, die je nach Erfordernis der zu kultivierenden Zellen aus unterschiedlichen Kunststoffmaterialien hergestellt sein kann, z.B. ebenfalls aus Polyethylenterephtaltat. Die Membran trägt dabei die Zellkultur.

Die Aufnahmen 112 sind in ihrem Bodenbereich mit einem gemeinsamen Leitungssystem 114 verbunden, das wiederum in zwei Anschlußstutzen 116 verzweigt, an die eine Flüssigkeitspegel-Steuereinheit angeschlossen ist, mit der die Zellkulturen in den Kulturbehältern 112 mit Flüssignährstoff versorgt werden können (z.B. eine pulsmäßige Steuerung von Zufuhr und Abfuhr der Nährflüssigkeit). Die nicht näher dargestellte Steuereinheit steuert das Niveau des Flüssigmediums innerhalb der Kulturbehälter 112. Damit können beispielsweise die Zellkulturen innerhalb der Kulturbehälter 112 periodisch abwechselnd basal und submers ernährt werden, indem der Flüssigkeitspegel der Nährflüssigkeit entsprechend oberhalb oder unterhalb der Oberfläche der Zellkulturen eingestellt wird. Für weitere Details bezüglich der Pulssteuerung und Niveauregelung des flüssigen Mediums innerhalb des Kulturbehälters 112 wird ebenfalls auf die oben genannte Patentanmeldung verwiesen. Der Untersatz 108 ist wiederum als flüssigkeitsdichter Hohlkörper ausgebildet, mit einem Flüssigkeitszulauf 118 und einem Flüssigkeitsablauf 120, über die eine temperierbare Flüssigkeit zum Temperieren der im Kulturbehälter 112 aufgenommenen Zellkulturen durch den Untersatz 108 geleitet werden kann. Die Aufnahmen 110 bzw. Kulturbehälter 112 sind dabei ebenfalls flüssigkeitsdicht gegen den genannten Innenraum abgedichtet.

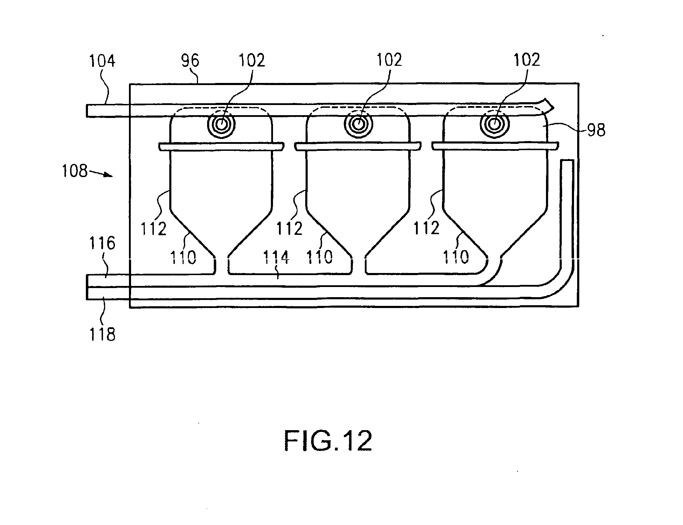

Fig. 12 zeigt eine Variante einer zusammenbaubaren Kultur/Expositionsvorrichtung, bei der der Untersatz 108 der Fig. 11 mit dem Aufsatz 96 der Fig. 10 kombiniert ist. Der Bausatz ist so gestaltet, daß auch die Unter- 2 und Aufsätze 4 entsprechend mit den Unter- 108 und Aufsätzen 96 beliebig kombinierbar sind, je nach dem, welche Kulturen untersucht werden sollen (für Zellkulturen wird bevorzugt der Untersatz 108 verwendet, für Prokaryonten-Kulturen wird bevorzugt der Untersatz 2 verwendet, wobei diese Kulturen dann zwecks Ernährung in einer geeigneten Substanz, z.B. Agar in den Kulturbehältern gehalten werden) und ob eine zeitlich und räumlich möglichst homogene Verteilung des zu beaufschlagenden gasförmigen Mediums Voraussetzung für die Untersuchung ist (falls dies untersuchungskritisch ist, wird der Aufsatz 4 mit der erfindungsgemäßen Strömungsführung verwendet, wenn dies weniger untersuchungskritisch ist, wird bevorzugt der Aufsatz 98 verwendet).

Insgesamt kann die Kultur/Expositionsvorrichtung so ausgebildet sein, daß sie auf einer automatischen Bestückungsstraße durch Roboter mit den entsprechenden Kulturbehältern 10 bzw. 112 und den darin aufgenommenen Kulturen (Eukaryonten- oder Prokaryonten-Kulturen) bestückt werden kann. Hierzu ist beispielsweise anstelle des in Fig. 1 gezeigten Verschlusses 6 in Form eines Schnallenmechanismuses ein robotertauglicher Verschluß vorgesehen, mit dem Unter-2, 108 und Aufsatz 4, 96 durch einen Roboter leicht geöffnet und geschlossen werden können. Ferner sind an den Kulturbehältern 10 bzw. 112 beispielsweise Mittel vorgesehen (Magnete, Einkerbungen, Rastnasen, etc.), mit denen ein Roboterarm die Kulturbehälter 10 bzw. 112 leicht innerhalb der Aufnahmen 8 bzw. 110 greifen und herausnehmen kann bzw. entsprechend dort einsetzen kann. Schließlich ist die Kultur/Expositionseinrichtung insgesamt so ausgebildet, daß sie in einer Spülstation, nachdem beispielsweise alle Kulturbehälter 10 bzw. 112 entnommen wurden, leicht gespült werden kann. Es werden also möglichst Kanten und sonstige schwer zugängliche Zwischenräume, die in Kontakt mit dem zu beaufschlagenden gasförmigen Medium und den Kulturen gelangen, vermieden.

Insbesondere können erfindungsgemäß mit der in Fig. 1 gezeigten Kultur/Expositionsvorrichtung (wobei der Aufsatz 4 durch den Aufsatz 96 ausgewechselt werden kann, wenn eine entsprechend homogene Strömungsverteilung nicht erforderlich ist) Prokaryonten (z.B. Bakterien, Pilze, usw.) kultiviert und mit einem gasförmigen Medium beaufschlagt werden, was völlig neuartige Untersuchungsmöglichkeiten mit dieser Art von Kulturen eröffnet. Bislang wurden solche Untersuchungen nur an Zellkulturen durchgeführt. So läßt sich beispielsweise der Ames-Test, der bislang nur mit Flüssigwirkstoffen (die zusammen mit den Bakterien in Agar eingekapselt wurden) durchgeführt wurde, auf mit dem gasförmigen Medium mitgeführte Wirkstoffe (das gasförmige Medium selbst oder darin enthaltene Flüssig- und/oder Feststoffe) erweitern (die Bakterien werden ggf. nur noch mit ihren Nährsubstanzen in Agar eingekapselt und der Wirkstoff strömt über die eingekapselten Bakterien).

Der Volumenstrom des beaufschlagenden gasförmigen Mediums wird bei solchen Untersuchungen gewöhnlich auf folgende Werte eingestellt, etwa 80 ml/Minute, etwa 50 ml/Minute, etwa 10 ml/Minute, so daß die Strömung innerhalb der Strömungsführungen im Bereich niedriger Reynoldszahlen liegt und als schleichende Strömung klassifiziert werden kann. Zudem werden beispielsweise bei Untersuchungen der Auswirkungen von Tabakrauch auf Lungenzellen Rauch/Luft-Mischungsverhältnisse von 1:5 bis 1:10 eingestellt, und diese Luft/Rauch-Mischung bei einer Temperatur von etwa 35° Celsius gehalten (beispielsweise durch die entsprechenden Heizmittel um die Strömungsführungen 40 bzw. 98). Von dem in der Luft mitgeführten Rauch werden erfahrungsgemäß etwa 1% von den Lungenzellen gebunden.

## Patentansprüche

1. Kultur/Expositionsvorrichtung zum Aufnehmen von Kulturen (72) mit einer Einrichtung zum Beaufschlagen der aufgenommenen Kultur (72) mit einem gasförmigen Medium, die eine mechanische Strömungsführung (40) mit einem Eingang (42) zum Einleiten des gasförmigen Mediums in die Strömungsführung (40) und einer oberhalb der Oberfläche der Kultur (72) mündenden Ausgangsmündung (44) umfaßt, wobei die Strömungsführung (40) das gasförmige Medium zwangsführt, **dadurch gekennzeichnet, daß** die Innenfläche der Ausgangsmündung (44) der Strömungsführung (40) sich in Strömungsrichtung trompetenförmig öffnet.

2. Kultur/Expositionsvorrichtung nach Anspruch 1, bei der die Innenfläche der Strömungsführung (40) quer zur Strömungsrichtung rotationssymmetrisch, insbesondere hyperboloidförmig, ist.

3. Kultur/Expositionsvorrichtung nach einem der vorhergehenden Ansprüche, bei der der Abstand der Ausgangsmündung (44) zur Oberfläche der Kultur (72) derart eingestellt ist, daß der Durchflußquerschnitt (Q2) des Ringspalts zwischen Mündungskante (48) der Strömungsführung (40) und Oberfläche der Kultur (72) kleiner als der Durchflußquerschnitt (Q1) in der Strömungsführung (40) stromauf der sich trompetenförmig öffnenden Ausgangsmündung (44) ist.

4. Kultur/Expositionsvorrichtung nach Anspruch 3, mit einer Einstelleinrichtung zum Verstellen des Abstandes.

5. Kultur/Expositionsvorrichtung nach einem der vorhergehenden Ansprüche, mit einem schalenförmigen Kulturbehälter (10; 112) zur Aufnahme von Kulturen (72), wobei das Verhältnis zwischen Innendurchmesser des Kulturbehälters (10) in Höhe der Mündungskante (48) der Ausgangsmündung (44) und Außendurchmesser der Ausgangsmündung (44) derart dimensioniert ist, daß der Durchflußquerschnitt (Q3) des Ringspalts zwischen Mündungskante (48) und Innenwand des Kulturbehälters (10; 112) größer als der Durchflußquerschnitt (Q1) des Ringspalts in der Strömungsführung (40) stromauf der sich trompetenförmig öffnenden Ausgangsmündung (44) ist.

6. Kultur/Expositionsvorrichtung nach einem der vorhergehenden Ansprüche, die einen Innenraum (8) zur Aufnahme des Kulturbehälters (10; 112) mit der Kultur (72) mit einem die Strömungsführung (40) tragenden ersten Raumbereich (50) und einem den Kulturbehälter (10; 112) tragenden zweiten Raumbereich (8) umfaßt, wobei die Strömungsführung (40) durch einen zentralen Abschnitt (52) des oberen Raumbereichs (50) in den Innenraum (8, 50) ragt und im verbleibenden Abschnitt des oberen Raumbereichs (50) eine Vakuumöffnung (68) vorgesehen ist, die mit einem Mittel zum Erzeugen eines Vakuums verbunden ist.

7. Kultur/Expositionsvorrichtung nach Anspruch 6, bei der die Beaufschlagungseinrichtung eine luftdicht zwischen Innenwand des Innenraums (8, 50) und Außenwand der Strömungsführung (40) angeordnete Ringblende (62) mit rotationssymmetrisch verteilten Luftdurchlässen (66) umfaßt.

8. Kultur/Expositionsvorrichtung nach Anspruch 7, bei der über die Dimensionierung der Luftdurchlässe (66) der Ringblende (62) der Unterdruck in der Beaufschlagungseinrichtung kalibriert wird.

9. Kultur/Expositionsvorrichtung nach einem der vorhergehenden Ansprüche, bei der die Beaufschlagungseinrichtung einen Volumen- und/oder Massendurchflußmesser umfaßt und ein mit dem Volumen- und/oder Massendurchflußmesser verbundenes Stellventil.

10. Kultur/Expositionsvorrichtung nach einem der vorhergehenden Ansprüche, bei der die Beaufschlagungseinrichtung mit einer Heizeinrichtung zum Temperieren des zuzuführenden gasförmigen Mediums ausgestattet sind, die an der Wand der Strömungsführung (40) angeordnete Heizmittel umfaßt.

11. Kultur/Expositionsvorrichtung nach einem der vorhergehenden Ansprüche, die zur Aufnahme mehrerer Kulturbehälter (10; 112) ausgestaltet ist, wobei die Beaufschlagungseinrichtung je eine Strömungsführung (40) pro Kulturbehälter (10; 112) umfaßt, deren Eingänge (42) mit einem gemeinsamen Ansaugstutzen zum Einleiten des gasförmigen Mediums in die Beaufschlagungseinrichtung verbunden sind.

12. Kultur/Expositionsvorrichtung nach einem der vorhergehenden Ansprüche, die zur Aufnahme eines Kulturbehälters (10) zur Aufnahme von Prokaryonten-Kulturen ausgebildet ist, einen Untersatz (2) entsprechend zur Aufnahme dieses Kulturbehälters (10) und einer Heizeinrichtung (36, 38) zum Temperieren des Kulturbehälters (10) umfaßt.

13. Kultur/Expositionsvorrichtung nach Anspruch 11 oder 12, die eine Auswurfeinrichtung (12) zum Auswerfen der Kulturbehälter (10) umfaßt.

14. Kultur/Expositionsvorrichtung nach einem der vorhergehenden Ansprüche, die zur Aufnahme eines Kulturbehälters (112) zur Aufnahme von Zellkulturen ausgebildet ist, und einen Untersatz (108) entsprechend zur Aufnahme dieses Kulturbehälters (112) und eine Versorgungseinheit zum Versorgen der Zellkultur im Kulturbehälter (112) mit einem flüssigen Medium umfaßt.

15. Kultur/Expositionsvorrichtung nach Anspruch 14, bei der die Versorgungseinheit zum wahlweisen submersen oder basalen Versorgen der Zellkultur mit dem flüssigen Medium ausgebildet ist.

16. Kultur/Expositionsvorrichtung nach Anspruch 14 oder 15, bei der die Versorgungseinheit ein Heizmittel (118, 120) zum Temperieren des flüssigen Mediums aufweist.

17. Kultur/Expositionsvorrichtung nach einem der Ansprüche 12 bis 16, die zweiteilig aus dem den Kulturbehälter (10; 112) aufnehmenden Untersatz (2; 108) und einem die Beaufschlagungseinrichtung aufnehmenden Aufsatz (4) aufgebaut ist und durch Trennen von Unter- (2; 108) und Aufsatz (4) Zugriff auf den Kulturbehälter (10; 112) gewährt, bei der Auf- (4) und Untersatz (2) derart ausgebildet sind, daß ein Aufsatz (4) mit der Beaufschlagungseinrichtung sowohl in Verbindung mit einem Untersatz (2) nach Anspruch 12 oder 13 als auch in Verbindung mit einem Untersatz (108) nach einem der Ansprüche 14 bis 16 verwendet werden kann.

18. Kultur/Expositionsvorrichtung zum Aufnehmen von Kulturen mit einer Einrichtung zum Beaufschlagen der aufgenommenen Kultur (72) mit einem gasförmigen Medium, die einen Ansaugstutzen (74, 74') mit einer Ansaugmündung (76) zum Ansaugen des gasförmigen Mediums und einem Ausgang (84; 88) aufweist, der mit einer Strömungsführung zum Führen des gasförmigen Mediums bis oberhalb der Oberfläche der Kultur (72) verbunden ist, **dadurch gekennzeichnet, daß** die Innenfläche der Ansaugmündung (76) des Ansaugstutzens (74; 74') sich gegen die Strömungsrichtung trompetenförmig öffnet.

19. Kultur/Expositionsvorrichtung nach Anspruch 18, bei der die Innenfläche des Ansaugstutzen (74; 74') quer zur Strömungsrichtung rotationssymmetrisch, insbesondere hyperboloidförmig, ist.

20. Kultur/Expositionsvorrichtung nach Anspruch 18 oder 19, bei der die Ansaugmündung (76) mit einer gasdurchlässigen Abdeckung (82) aus einem hochporösen Schaumstoff abgedeckt ist.

21. Kultur/Expositionsvorrichtung nach einem der Ansprüche 18 bis 20, bei der der Ausgang (84; 88) des Ansaugstutzens (74; 74') eine Verzweigung zu mehreren Strömungsführungen umfaßt, die am stromab gelegenen Ende des Führungsabschnitt (78) mit zylindrischer Innenfläche angeordnet ist.

22. Kultur/Expositionsvorrichtung nach Anspruch 21, bei der die Verzweigung radial von der Außenwand des Führungsabschnitts (78) mit zylindrischer Innenfläche abstehende Anschlußstutzen (84) aufweist.

23. Kultur/Expositionsvorrichtung nach Anspruch 21, bei der die Verzweigung in Längsrichtung vom Führungsabschnitt (78) mit zylindrischer Innenfläche von dessen Bodenbereich abstehende Anschlußstutzen (88) aufweist.

24. Kultur/Expositionsvorrichtung nach Anspruch 22 oder 23, bei der innerhalb des Führungsabschnitts (78) mit zylindrischer Innenfläche in dessen Längsrichtung verlaufende, von dessen Boden bis über die Höhe der Anschlußstut-zen (84; 88) verlaufende Trennwände (86; 94) angeordnet sind, welche getrennte Ansaugräume für jeden Anschluß-stutzen (84; 88) bilden, die derart dimensioniert sind, daß das Ansaugvolumen in alle Ansaugstutzen (84; 88) bei gleicher Ansaugleistung in etwa gleich groß ist.

25. Bausatz für den Zusammenbau einer Kultur/Expositionsvorrichtung nach einem der vorhergehenden Ansprüche, der folgende vier Bausatzelemente enthält:
- Aufsätze (4) nach einem der Ansprüche 17 bis 24,
- Aufsätze (96) mit einer Einrichtung zum Beaufschlagen der in einem Kulturbehälter (10; 112) aufgenommenen Kultur (72) mit einem gasförmigen Medium, wobei die Beaufschlagungseinrichtung einen Ansaugstutzen zum Ansaugen des gasförmigen Mediums und eine mit dem Ansaugstutzen verbundene Strömungsführung (98) zum trompetenförmigen Führen des gasförmigen Mediums bis oberhalb der Oberfläche der Kultur (72) umfaßt,
- Untersätze (2) nach Anspruch 12 oder 13, sowie
- Untersätze (108) nach einem der Ansprüche 14 bis 16.

26. Verfahren zur Kultivierung und Exposition von Prokaryonten unter Verwendung einer Kultur/Expositionsvorrichtung mit einer Aufnahme (8) zum Aufnehmen eines Kulturbehälters (10) mit den zu kultivierenden Prokaryonten sowie einer Einrichtung zum Beaufschlagen der im Kulturbehälter aufgenommenen Prokaryonten mit einem gasförmigen Medium, wobei die Beaufschlagungseinrichtung einen Ansaugstutzen zum Ansaugen des gasförmigen Mediums und eine mit dem Ansaugstutzen verbundene Störmungsführung (40; 98) zum trompetenförmigen Führen des gasförmigen Mediums über die im Kulturbehälter (10; 112) aufgenommenen Prokaryonten umfaßt.

27. Kultur/Expositionsvorrichtung zum Durchführen eines Verfahrens nach Anspruch 26, mit einer Aufnahme (8) zum Aufnehmen eines Kulturbehälters (10) mit den zu kultivierenden Prokaryonten sowie einer Einrichtung zum Beaufschlagen der im Kulturbehälter aufgenommenen Prokaryonten mit einem gasförmigen Medium, wobei die Beaufschlagungseinrichtung einen Ansaugstutzen zum Ansaugen des gasförmigen Mediums und eine mit dem Ansaugstutzen verbundene Strömungsführung (40; 98) zum trompetenförmigen Führen des gasförmigen Mediums über die im Kulturbehälter (10; 112) aufgenommenen Prokaryonten umfaßt.

## Claims

1. Culture/exposure device for accommodating cultures (72) with a system for charging the accommodated culture (72) with a gaseous medium and comprising a mechanical flow guide (40) with an entry (42) for introducing the gaseous medium into the flow guide (40) and an exit opening (44) which opens above the surface of the culture (72), the flow guide (40) forcibly guiding the gaseous medium, **characterized in that** the inner surface of the exit opening (44) of the flow guide (40) opens in a trumpet-like manner in the direction of flow.

2. Culture/exposure device according to claim 1, in which the inner surface of the flow guide (40) is rotationally symmetric, in particular hyperboloidal, at right angles to the direction of flow.

3. Culture/exposure device according to one of the preceding claims, in which the distance from the exit opening (44) to the surface of the culture (72) is established such that the flow cross-section (Q2) of the annular gap between the opening edge (48) of the flow guide (40) and the surface of the culture (72) is smaller than the flow cross-section (Q1) in the flow guide (40) upstream of the exit opening (44) which opens in a trumpet-like manner.

4. Culture/exposure device according to claim 3, with an adjustment system for adjusting the distance.

5. Culture/exposure device according to one of the preceding claims, with a dish-shaped culture container (10; 112) for accommodating cultures (72), wherein the ratio between the internal diameter of the culture container (10) at the level of the opening edge (48) of the exit opening (44) and the external diameter of the exit opening (44) has dimensions such that the flow cross-section (Q3) of the annular gap between the opening edge (48) and the internal wall of the culture container (10; 112) is greater than the flow cross-section (Q1) of the annular gap in the flow guide (40) upstream of the exit opening (44) which opens in a trumpet-like manner.

6. Culture/exposure device according to one of the preceding claims, which comprises an inner chamber (8) for accommodating the culture container (10; 112) with the culture (72), with a first chamber region (50) which carries the flow guide (40) and a second chamber region (8) which carries the culture container (10; 112), the flow guide (40) protruding through a central section (52) of the upper chamber region (50) into the inner chamber (8, 50), and a vacuum opening (68) which is connected to a means for generating a vacuum being provided in the remaining section of the upper chamber region (50).

7. Culture/exposure device according to claim 6, in which the charging system comprises an annular diaphragm (62) which has rotationally symmetrically distributed air passages and is arranged in an air-tight manner between the internal wall of the inner chamber (8, 50) and the external wall of the flow guide (40).

8. Culture/exposure device according to claim 7, in which the reduced pressure in the charging system is calibrated via the dimensions of the air passages (66) of the annular diaphragm (62).

9. Culture/exposure device according to one of the preceding claims, in which the charging system comprises a volume and/or mass flow meter and a control valve connected to the volume and/or mass flow meter.

10. Culture/exposure device according to one of the preceding claims, in which the charging system is equipped with a heating system for temperature control of the gaseous medium to be fed in, which comprises a heating means arranged on the wall of the flow guide (40).

11. Culture/exposure device according to one of the preceding claims, which is configured for accommodation of several culture containers (10; 112), wherein the charging system in each case comprises a flow guide (40) per culture container (10; 112), the entries (42) of which are connected to a common intake tube for introducing the gaseous medium into the charging system.

12. Culture/exposure device according to one of the preceding claims, which is constructed for accommodation of a culture container (10) for accommodation of prokaryotic cultures, a base (2) correspondingly for accommodation of this culture container (10) and a heating system (36, 38) for temperature control of the culture container (10).

13. Culture/exposure device according to claim 11 or 12, which comprises an ejector system (12) for ejecting the culture containers (10).

14. Culture/exposure device according to one of the preceding claims, which is constructed for accommodation of a culture container (112) for accommodation of cell cultures, and comprises a base (108) correspondingly for accommodation of this culture container (112) and a supply unit for supplying the cell culture in the culture container (112) with a liquid medium.

15. Culture/exposure device according to claim 14, in which the supply unit is constructed for optional submersed or basal supplying of the cell culture with the liquid medium.

16. Culture/exposure device according to claim 14 or 15, in which the supply unit has a heating means (118, 120) for temperature control of the liquid medium.

17. Culture/exposure device according to one of claims 12 to 16, which is constructed in two parts from the base (2; 108) accommodating the culture container (10; 112) and a top (4) accommodating the charging system, and by separation of the base (2; 108) and top (4) allows access to the culture container (10; 12), in which the top (4) and base (2) are constructed such that a top (4) with the charging system can be used both in combination with a base (2) according to claim 12 or 13 and in combination with a base (108) according to one of claims 14 to 16.

18. Culture/exposure device for accommodating cultures with a system for charging the accommodated culture (72) with a gaseous medium, which comprises an intake tube (74, 74') with an intake opening (76) for drawing in the gaseous medium and an exit (84; 88) which is connected to a flow guide for guiding the gaseous medium to above the surface of the culture (72), **characterized in that** the inner surface of the intake opening (76) of the intake tube (74, 74') opens in a trumpet-like manner counter to the direction of flow.

19. Culture/exposure device according to claim 18, in which the inner surface of the intake tube (74; 74') is rotationally symmetric, in particular hyperboloidal, at right angles to the direction of flow.

20. Culture/exposure device according to claim 18 or 19, in which the intake opening (76) is covered with a gas-permeable cover (82) of a highly porous foam.

21. Culture/exposure device according to one of claims 18 to 20, in which the exit (84; 88) of the intake tube (74; 74') comprises a junction to a plurality of flow guides which is arranged at the downstream end of the guide section (78) with a cylindrical inner surface.

22. Culture/exposure device according to claim 21, in which the junction comprises connection tubes (84) standing out radially from the external wall of the guide section (78) with a cylindrical inner surface.

23. Culture/exposure device according to claim 21, in which the junction comprises connection tubes (88) standing out in the longitudinal direction from the guide section (78) with a cylindrical inner surface, from the base region thereof.

24. Culture/exposure device according to claim 22 or 23, in which within the guide section (78) with a cylindrical inner surface there are arranged dividing walls (86; 94) which run in the longitudinal direction thereof and run from the base thereof to above the level of the connection tubes (84; 88), and form separate intake chambers for each connection tube (84; 88), the dimensions of which are such that the intake volume is approximately the same in all the intake tubes (84; 88) at the same intake rate.

25. Kit for assembling a culture/exposure device according to one of the preceding claims, which comprises the following four kit elements:
- tops (4) according to one of claims 17 to 24,
- tops (96) with a system for charging the culture (72) accommodated in a culture container (10; 112) with a gaseous medium, wherein the charging system comprises an intake tube for drawing in the gaseous medium and a flow guide (98) connected to the intake tube for guiding the gaseous medium in a trumpet-like manner to above the surface of the culture (72),
- bases (2) according to claim 12 or 13 and
- bases (108) according to one of claims 14 to 16.

26. Method for cultivating and exposing prokaryotes using a culture/exposure device with a receptacle (8) for accommodating a culture container (10) with the prokaryotes to be cultivated and a system for charging the prokaryotes accommodated in the culture container with a gaseous medium, wherein the charging system comprises an intake tube for drawing in the gaseous medium and a flow guide (40; 98) connected to the intake tube for guiding the gaseous medium in a trumpet-like manner over the prokaryotes accommodated in the culture container (10; 112).

27. Culture/exposure device for implementing a method according to claim 26, with a receptacle (8) for accommodating a culture container (10) with the prokaryotes to be cultivated and a system for charging the prokaryotes accommodated in the culture container with a gaseous medium, wherein the charging system comprises an intake tube for drawing in the gaseous medium and a flow guide (40; 98) connected to the intake connector for guiding the gaseous medium in a trumpet-like manner over the prokaryotes accommodated in the culture container (10; 112).

## Revendications

1. Dispositif de culture/d'exposition pour recevoir des cultures (72) avec un dispositif destiné à soumettre la culture reçue (72) à un fluide gazeux, comprenant un guide d'écoulement mécanique (40) avec une entrée (42) pour l'introduction du fluide gazeux dans le guide d'écoulement (40) et une embouchure de sortie (44) débouchant au-dessus de la surface de la culture (72), le guide d'écoulement (40) guidant le fluide gazeux de manière forcée, **caractérisé par le fait que** la surface intérieure de l'embouchure de sortie (44) du guide d'écoulement (40) s'ouvre en forme de trompette dans le sens de l'écoulement.

2. Dispositif de culture/exposition selon la revendication 1, dans lequel la surface intérieure du guide d'écoulement (40) est symétrique en rotation transversalement au sens de l'écoulement, en particulier en forme d'hyperboloïde.

3. Dispositif de culture/exposition selon l'une des revendications précédentes, dans lequel la distance de l'embouchure de sortie (44) par rapport à la surface de la culture (72) est réglée de sorte que la section de passage (Q2) de l'interstice annulaire entre le bord de l'embouchure d'écoulement (48) du guide d'écoulement (40) et la surface de la culture (72) soit plus petite que la section de passage (Q1) dans le guide d'écoulement (40) en amont de l'embouchure de sortie (44) s'ouvrant en forme de trompette.

4. Dispositif de culture/exposition selon la revendication 3, avec un dispositif de réglage destiné à modifier la distance.

5. Dispositif de culture/exposition selon l'une des revendications précédentes, avec un récipient à culture en forme de coquille (10; 112) destiné à recevoir des cultures (72), le rapport entre le diamètre intérieur du récipient à culture (10) étant dimensionné en hauteur du bord (48) de l'embouchure de sortie (44) et en diamètre extérieur de l'embouchure de sortie (44) de sorte que la section de passage (Q3) de l'interstice annulaire entre le bord d'embouchure (48) et la paroi intérieure du récipient à culture (10; 112) soit plus grande que la section de passage (Q1) de l'interstice annulaire dans le guide d'écoulement (40) en amont de l'embouchure de sortie (44) débouchant en forme de trompette.

6. Dispositif de culture/exposition selon l'une des revendications précédentes, comprenant un espace intérieur (8) destiné à recevoir le récipient à culture (10; 112) avec la culture (72) avec une première zone d'espace (50) portant le guide d'écoulement (40) et une deuxième zone d'espace (8) portant le récipient à culture (10; 112), le guide d'écoulement (40) pénétrant à travers un segment central (52) de la zone d'espace supérieure (50) dans l'espace intérieur (8, 50), et dans le segment restant de la zone d'espace supérieure (50) étant prévue une ouverture à vide (68) qui est reliée à un moyen de génération de vide.

7. Dispositif de culture/exposition selon la revendication 6, dans lequel le dispositif de soumission comporte un diaphragme annulaire (62) disposé de manière étanche à l'air entre la paroi intérieure de l'espace intérieur (8, 50) et la paroi extérieure du guide d'écoulement (40), avec des passages d'air (66) répartis de manière symétrique en rotation.

8. Dispositif de culture/exposition selon la revendication 7, dans lequel par le dimensionnement des passages d'air (66) du diaphragme annulaire (62) est calibrée la dépression dans le dispositif de soumission.

9. Dispositif de culture/exposition selon l'une des revendications précédentes, dans lequel le dispositif de soumission comporte un dispositif de mesure de débit de volume et/ou de masse et une soupape de réglage connectée au dispositif de mesure de débit de volume et/ou de masse.

10. Dispositif de culture/exposition selon l'une des revendications précédentes, dans lequel le dispositif de soumission est muni d'un dispositif de chauffage destiné à thermostatiser le fluide gazeux à alimenter et qui comporte des moyens de chauffage disposés sur la paroi du guide d'écoulement (40).

11. Dispositif de culture/exposition selon l'une des revendications précédentes, qui est conçu pour recevoir plusieurs récipients à culture (10; 112), le dispositif de soumission comportant un guide d'écoulement (40) pour chaque récipient à culture (10; 112) dont les entrées sont reliées à une tubulure commune d'introduction du fluide gazeux dans le dispositif de soumission.

12. Dispositif de culture/exposition selon l'une des revendications précédentes, qui est conçu pour recevoir un récipient à culture (10) destiné à recevoir des cultures procaryotes et qui comporte un socle (2) correspondant destiné à recevoir ce récipient à culture (10) et un dispositif de chauffage (36, 38) destiné à thermostatiser le récipient à culture (10).

13. Dispositif de culture/exposition selon la revendication 11 ou 12, comportant un dispositif d'expulsion (12) destiné à expulser le récipient à culture (10).

14. Dispositif de culture/exposition selon l'une des revendications précédentes, qui est conçu pour recevoir un récipient à culture (112) destiné à recevoir des cultures cellulaires, et qui comporte un socle (108) correspondant destiné à recevoir ce récipient à culture (112) et une unité d'alimentation destinée à alimenter la culture cellulaire dans le récipient à culture (112) en fluide liquide.

15. Dispositif de culture/exposition selon la revendication 14, dans lequel l'unité d'alimentation est réalisée de manière à alimenter de manière immergée ou basale la culture cellulaire en fluide liquide.

16. Dispositif de culture/exposition selon la revendication 14 ou 15, dans lequel l'unité d'alimentation présente un moyen de chauffage (118, 120) destiné à thermostatiser le fluide liquide.

17. Dispositif de culture/exposition selon l'une des revendications 12 à 16, qui est construit en deux parties à partir du socle (2, 108) recevant le récipient à culture (10; 112) et un rehaussement (4) recevant le dispositif de soumission, et garantissant, par une séparation du socle (2; 108) et du rehaussement (4), un accès au récipient à culture (10, 112), dans lequel le rehaussement (4) et le socle (2) sont conçus de sorte qu'un rehaussement (4) avec le dispositif de soumission peut être utilisé tant en liaison avec un socle (2) selon la revendication 12 ou 13 qu'en liaison avec un socle (108) selon l'une des revendications 14 à 16.

18. Dispositif de culture/exposition pour recevoir des cultures avec un dispositif de soumission de la culture reçue (72) à un fluide gazeux, présentant une tubulure de succion (74, 74') avec une embouchure d'aspiration (76) destinée à aspirer le fluide gazeux et une sortie (84, 88) qui est reliée à un guide d'écoulement destiné à guider le fluide gazeux jusqu'au-dessus de la surface de la culture (72), **caractérisé par le fait que** la surface intérieure de l'embouchure d'aspiration (76) de la tubulure de succion (74, 74') s'ouvre en trompette dans le sens contraire à l'écoulement.

19. Dispositif de culture/exposition selon la revendication 18, dans lequel la surface intérieure de la tubulure de succion (74, 74') est symétrique en rotation transversalement au sens d'écoulement, en particulier en forme d'hyperboloïde.

20. Dispositif de culture/exposition selon la revendication 18 ou 19, dans lequel l'embouchure d'aspiration (76) est recouverte d'un couvercle perméable au gaz (82) en une mousse hautement poreuse.

21. Dispositif de culture/exposition selon l'une des revendications 18 à 20, dans lequel la sortie (84, 88) de la tubulure de succion (74; 74') comporte une ramification vers plusieurs guides d'écoulement qui est disposée à l'extrémité aval du segment de guidage (78) à surface intérieure cylindrique.

22. Dispositif de culture/exposition selon la revendication 21, dans lequel la ramification présente des tubulures de raccordement (84) distantes radialement de la paroi extérieure du segment de guidage (78) à surface intérieure cylindrique.

23. Dispositif de culture/exposition selon la revendication 21, dans lequel la ramification présente, dans le sens longitudinal du segment de guidage (78) à surface intérieure cylindrique, des tubulures de raccordement (88), distantes de la zone de fond de ce dernier.

24. Dispositif de culture/exposition selon la revendication 22 ou 23, dans lequel sont disposées, à l'intérieur du segment de guidage (78) à surface intérieure cylindrique, des cloisons (86; 94) s'étendant, dans sa direction longitudinale, du fond de ce dernier jusqu'au-dessus de la hauteur des tubulures de raccordement (84; 88) et qui forment des espaces d'aspiration séparés pour chaque tubulure de raccordement (84; 88) et qui sont dimensionnés de sorte que le volume d'aspiration est environ le même dans toutes les tubulures d'aspiration (84; 88) à puissance d'aspiration identique.

25. Kit de montage pour l'assemblage d'un dispositif de culture/exposition selon l'une des revendications précédentes, comportant les quatre éléments de kit de montage suivants:
- des rehaussements (4) selon l'une des revendications 17 à 24,
- des rehaussement (96) avec un dispositif de soumission de la culture (72) reçue dans un récipient à culture (10; 112) à un fluide gazeux, le dispositif de soumission comportant une tubulure de succion destinée à aspirer le fluide gazeux et un guide d'écoulement (98) relié à la tubulure de succion destiné à guider le fluide gazeux en forme de trompette jusqu'au-dessus de la surface de la culture (72),
- des socles (2) selon la revendication 12 ou 13, ainsi que
- des socles (108) selon l'une des revendications 14 à 16.

26. Procédé de culture et d'exposition de procaryotes à l'aide d'un dispositif de culture/exposition avec un dispositif de réception destiné à recevoir (8) un récipient à culture (10) avec les procaryotes à cultiver, ainsi qu'un dispositif de soumission des procaryotes reçus dans le récipient à culture à un liquide gazeux, le dispositif de soumission comportant une tubulure de succion destinée à aspirer le fluide gazeux et un guide d'écoulement (40; 98) relié à la tubulure d'aspiration destiné à guider le fluide gazeux en forme de trompette au-dessus des procaryotes reçus dans le récipient à culture (10; 112).

27. Dispositif de culture/d'exposition pour la mise en oeuvre d'un procédé selon la revendication 26, avec un dispositif de réception (8) destiné à recevoir un récipient à culture (10) avec les procaryotes à cultiver, ainsi qu'un dispositif de soumission des procaryotes reçus dans le récipient à culture à un liquide gazeux, le dispositif de soumission comportant une tubulure de succion destinée à aspirer le fluide gazeux et un guide d'écoulement (40; 98) relié à la tubulure d'aspiration destiné à guider le fluide gazeux en forme de trompette au-dessus des procaryotes reçus dans le récipient à culture (10; 112).
